# EUROPEAN PATENT SPECIFICATION

(11) **EP 4 580 721 B1**
(45) Date of publication and mention of the grant of the patent: **30.09.2026**
(21) Application number: 23762474.7
(22) Date of filing: 30.08.2023
(51) Int. Cl.: A61M 25/00

(54) **CATHETER FOR DELIVERY OF THERAPEUTIC LIQUID FORMULATION**
KATHETER ZUR ABGABE EINER THERAPEUTISCHEN FLÜSSIGEN FORMULIERUNG
CATHÉTER POUR L'ADMINISTRATION D'UNE FORMULATION LIQUIDE THÉRAPEUTIQUE

(30) Priority: 02.09.2022 EP 22193609
(43) Date of publication of application: 09.07.2025
(73) Proprietor: Universiteit Gent, 9000 Gent (BE); Vrije Universiteit Brussel, 1050 Brussel (BE)
(72) Inventor: RAHIMI GORJI, Mohammad, 9000 Gent (BE); GHORBANIASL, Ghader, 1050 Brussel (BE); DEBBAUT, Charlotte, 9000 Gent (BE); CEELEN, Wim, 9000 Gent (BE); AUBERT, David, 9000 Gent (BE)
(74) Representative: De Clercq & Partners
(86) International application number: PCT/EP2023/073744
(87) International publication number: WO 2024/047081

(56) References cited:
- EP-A2- 1 649 880
- WO-A1-2015/146651
- WO-A1-2021/016579
- WO-A1-2022/022962
- US-A- 5 425 723
- US-A1- 2003 181 887
- US-A1- 2011 282 264
- US-B1- 6 241 710

## Description

### Field of the invention

The present invention in is the field of medical catheters, in particular for infusion of a therapeutic liquid formulation.

### Background to the invention

Intraperitoneal drug delivery is used for chemotherapy in patients with intraperitoneal malignancies. Typically a catheter (*e.g*. Tenckhoff) having a single outlet is implanted deep in the pelvis, and the therapeutic liquid formulation (chemotherapy agent) is introduced that will contact and treat the intraperitoneal malignancies. However, the introduced therapeutic liquid formulation tends to settle in the lower portion of the peritoneal cavity, thereby, not treating malignancies in the upper surfaces of the peritoneal cavity. Traditionally, in order to treat upper surface intraperitoneal malignancies, a large volume of therapeutic liquid formulation is used to fill the cavity so that both lower and upper portions receive the therapeutic liquid formulation. The large volume, however, causes problems of distension which is highly uncomfortable for the patient, for instance, causing pain, problems with breathing and digestion. Because of this, the therapeutic liquid formulation is drained away after a period of time, and after a further period of time, the large volume of therapeutic liquid formulation is introduced again. Under the standard technique, the concentration of therapeutic agent in the therapeutic liquid formulation is high. In addition, the time gap between drainage and re-administration of the therapeutic liquid formulation can cause resistance to the therapeutic liquid formulation.

CA 2,179,635 (US5425723A) describes a catheter for even arterial liquid distribution, having concentric lumens. The inner tube is not supported within the outer tube, and the relative axial positions of the inner and outer tubes varies which affects flow distribution through its outlets. WO 2021/016579 A1 discloses a catheter having a proximal subassembly and a distal subassembly having an infusion lumen for infusing cerebrospinal fluid and a plurality of infusion openings in fluid communication with the infusion lumen. US2011/0282264A1 discloses a single lumen drainage catheter having a proximal end and a distal end, and a plurality of openings formed therein. The plurality of openings includes a first opening, a second opening, and a most proximal opening. The distal end of the drainage catheter is for insertion into a human brain. US2003/0181887A1 discloses a catheter with an elongated tube with a plurality of exit holes along an infusion section of the catheter, and co-axially arranged elongated flexible porous member residing within the tube and forming
an annular space between the tube and the member. EP1649880A2 discloses a single lumen catheter for elution of drug. WO2022/022962A1 discloses a single lumen catheter for drainage of fluids or wound secretion. US6241710B1 discloses a surgical with a porous distal portion from which a liquid injectate weeps or oozes multidirectionally under injection pressure while the porous distal portion of the needle is inserted into a body surface. WO2015/146651A1 discloses a peritoneal catheter through which an endoscope can be passed easily.

Provided here is a device and a not claimed method to overcome the problems of the prior art

### Summary of the invention

Provided herein is a catheter (100) for intraperitoneal drug delivery having a proximal end (10) and a distal end (12) for distribution of a liquid outflow, the catheter (100) comprising:
- a first longitudinal lumen (120) disposed with a plurality of outlets (122a to f) in a flow distribution region (130) for passage of the liquid outflow,
- a distribution inlet (110) to the first lumen (120) disposed within a longitudinal central region (132) of the flow distribution region (130),
- a second longitudinal lumen (140), wherein the second lumen (140) is in fluid connection with the first lumen (120) at the distribution inlet (110), and is provided at a proximal end (12) with a catheter inlet (142) for inflow of the liquid,
- the first (120) and second (140) lumens are comprised in a tube (160) wherein a lumen (162) of the tube is partitioned into the first (120) and second (140) lumens by a partitioning wall (150) attached to the tube lumen wall (164),
- the outlets (122a to c) of the plurality on the distal (12) side of the distribution inlet (110) are longitudinally positioned and sized in mirrored symmetry with the plurality of outlets (122d to f) on the proximal (10) side of the distribution inlet (110),
- the distal (12) terminal end and proximal (10) terminal end of the first (120) lumen are liquid sealed closed,
- the distal (12) terminal end of the second (140) lumen is liquid sealed closed,
- the proximal (10) terminal end of the second (140) lumen is open and forms the catheter inlet (142) for inflow of the liquid,
wherein the plurality of outlets (122a to f) is configured to counteract a reduction in outflow as the distance from the distribution inlet (110) increases in the longitudinal direction (L).

Further provided herein but not currently claimed independently is a catheter (100) having a proximal end (10) and a distal end (12) for distribution of a liquid outflow, the catheter (100) comprising:
- a first longitudinal lumen (120) disposed with a plurality of outlets (122a to f) in a flow distribution region (130) for passage of the liquid outflow,
- a distribution inlet (110) to the first lumen (120) disposed within a longitudinal central region (132) of the flow distribution region (130),
- a second longitudinal lumen (140), wherein the second lumen (140) is in fluid connection with the first lumen (120) at the distribution inlet (110), and is provided at a proximal (10) end with a catheter inlet (142) for inflow of the liquid,
wherein the plurality of outlets (122a to f) is configured to counteract a reduction in outflow as the distance from the distribution inlet (110) increases in the longitudinal direction (L).

The quantity of outlets (122d to f) on the proximal (10) side of the distribution inlet (110) may be equal to the quantity of outlets (122a to c) on the distal (12) side of the distribution inlet (110) ±0, 1, or 2.

The longitudinal central region (132) of the flow distribution region (130):
- may have a length that is no more than 50% of the length of the flow distribution region (130), and
- may have a midpoint (M) that coincides with a midpoint of the longitudinal flow distribution region (130).

The catheter (100) may be disposed with at least one fixation element, configured to co-operate with a suture for suturing the catheter (100) to a structure within the body.

Each and every outlet (122a to f) of the plurality may be separated from the distribution inlet (110) in a longitudinal direction (L).

The size of each and every outlet (122d to f) of the plurality on the proximal (10) side of the distribution inlet (110) may be different, and the size may increase as function of longitudinal distance from the distribution inlet (110); and
- the size of each and every outlet (122a to c) of the plurality on the distal (12) side of the distribution inlet (110) may different, and the size may increase as function of longitudinal distance from the distribution inlet (110).

A distance between neighbouring pairs of outlets (122d-e, 122e-f) of the plurality on the proximal (10) side of the distribution inlet (110) may decrease as function of longitudinal distance of the neighbouring pairs from the distribution inlet (110); and
- a distance between neighbouring pairs of outlets (122a-b, 122b-c) of the plurality on the distal (12) side of the distribution inlet (110) may decrease as function of longitudinal distance of the neighbouring pairs from the distribution inlet (110).

The position and size of each and every outlet (122a to f) may be determined according to a Gaussian quadrature rule, wherein:
- the Gaussian quadrature rule defines, for each point of a Gauss point set of *n* Gauss points, a Gauss weight and a Gauss position, and
- the quantity of outlets of the catheter (100) corresponds to *n,* the Gauss positions of the Gauss point set of *n* Gauss points correspond to the positions of the outlets (122a to f), and the Gauss weights of the Gauss point set of *n* Gauss points correspond to the size of the outlets.

The catheter is preferably an implantable catheter wherein at least the flow distribution region (130) is implantable.

According to the present invention,
- the catheter (100) is for intraperitoneal drug delivery;
- the first (120) and second (140) lumens are comprised in a tube (160) wherein a lumen (162) of the tube is partitioned into the first (120) and second (140) lumens by a partitioning wall (150) attached to the tube lumen wall (164);
- the outlets (122a to c) of the plurality on the distal (12) side of the distribution inlet (110) are longitudinally positioned and sized in mirrored symmetry with the plurality of outlets (122d to f) on the proximal (10) side of the distribution inlet (110);
- the distal (12) terminal end and proximal (10) terminal end of the first (120) lumen are liquid sealed closed;
- the distal (12) terminal end of the second (140) lumen is liquid sealed closed;
- the proximal (10) terminal end of the second (140) lumen is open and forms the catheter inlet (142) for inflow of the liquid.

The distribution inlet (110) may be disposed on a connecting piece (300), wherein the connecting piece (300) comprises a body (302) having:
- a proximal (10) end coupling (312) for connection to a proximal portion (120,p) of the first longitudinal lumen (120), and for connection to a proximal portion (140,p) of the second longitudinal lumen (140), and
- a distal (12) end coupling (310) for connection to a distal portion (120,d) of the first longitudinal lumen (120), and for connection to a distal portion (140,d) of the second longitudinal lumen (140), and
wherein the connecting piece (300) is configured to:
- fluidly connect:
   - the proximal portion (120,p) of the first longitudinal lumen (120) to the distal portion (120,d) of the first longitudinal lumen (120), and
   - the proximal portion (140,p) of the second longitudinal lumen (140) to the distal portion (140,d) of the second longitudinal lumen (140),
- fluidly isolate the first and second longitudinal lumens (120, 140) from each other except through the distribution inlet (110) disposed on the connecting piece (300).

A catheter assembly (200) is provided herein comprising:
- a distribution hub (210) having a hub inlet (212) for inlet of liquid flow, and a plurality of hub outlets (214a to f) for a plurality of liquid outflows, where the distribution hub (210) is configured to split the inlet liquid flow into the plurality of liquid outflows, and
- a plurality of catheters (100a to 100f) as described herein, each operatively connected to a separate hub outlet (214a to f).

The distribution hub may comprise a body (216) containing a chamber (218) for holding the liquid and containing a plurality conduits (215a to f),
- each and every conduit (215a to f) may be fluidly connected at one end to the chamber (218), and at its other end to its respective hub outlet (214a to f),
- the hub inlet (212) may be fluidly connected to the chamber (218) via an inlet conduit (213).

The distribution hub (210) may be disposed with at least one fixation element, configured to co-operate with a suture for suturing the distribution hub (210) to a structure within the body.

Provided herein is a system comprising:
- a catheter (100), or a catheter assembly (200) as described herein,
- a reservoir containing the liquid that is a therapeutic liquid formulation, and
- a flow inducer configured to induce a flow of therapeutic liquid formulation from the reservoir to the catheter (100) or catheter assembly (200).

### Figure Legends

**FIG. 1** is a schematic longitudinal cross-sectional representation of a catheter described herein, wherein the first and second lumens are parallel aligned at the distal terminal end, and the second lumen extends past the first lumen in a proximal direction.
**FIG. 2** is a schematic longitudinal cross-sectional representation of a catheter described herein, wherein the second lumen and the first lumen have approximately the same longitudinal length.
**FIG. 2A** is a schematic transverse cross-sectional representation of the catheter of FIG. 2 at section a-a', without the partitioning wall (150).
**FIG. 2B** is a schematic transverse cross-sectional representation of the catheter of FIG. 2 at section a-a', with the partitioning wall (150).
**FIG. 3** is a schematic longitudinal cross-sectional representation of a catheter described herein, wherein the first and second lumens are partly disposed within a connecting piece, and the distribution inlet is disposed on the connecting piece.
**FIG. 4** is a schematic longitudinal cross-sectional representation of a connecting piece described herein.
**FIG. 5** is a schematic plan view representation of a distribution hub described herein.
**FIG. 6** is a schematic plan view representation of a distribution hub described herein, showing the interior chamber and conduits.
**FIG. 7** is a schematic plan view representation of a distribution hub described herein, wherein each hub outlet is operatively connected to a separate catheter.
**FIG. 8** is a graph showing outflow from different outlets of a catheter of the invention (solid line, solid circles) and from a CFD simulation of catheter of the invention (dashed line, open squares).
**FIG. 9** is a graph showing outflow from different outlets of a CFD simulation of catheter of the invention (dashed line, open squares), and from a CFD simulation of catheter not of the invention (dashed-dotted line, open triangles).
**FIG. 10** is a plan view of a catheter described herein showing a distribution of outlets of the first lumen, wherein the distal-most outlet (122c) and the proximal-most outlet (122f) of the flow distribution region (130) oblong shape.
**FIG. 11** is a graph showing outflow from different outlets of a CFD simulation of catheter of the invention having all-circular outlets (dashed line, open squares), and from a CFD simulation of catheter having a shape and distribution of outlets as shown in FIG. 11 (solid line, open circles).
**FIG. 12** is 3D representation of a distribution hub described herein, showing the interior chamber and conduits.

### Detailed description of invention

Before the present system and method of the invention are described, it is to be understood that this invention is not limited to particular devices (catheter, catheter assembly, distribution hub) and methods or combinations described, since such devices and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g*., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention. The following detailed description, therefore, is not to be taken in a limiting sense, and the scope of the present invention is defined by the appended claims.

The terms "distal" or "distal to" and "proximal" or "proximal to" are used throughout the specification, and are terms generally understood in the field to mean towards (proximal) or away (distal) from the operator's side of device (*e.g*. catheter, catheter assembly, distribution hub). Thus, "proximal" or "proximal to" means towards the operator's side and, therefore, away from the patient's side. Conversely, "distal" or "distal to" means towards the patient's side and, therefore, away from the operator's side.

The catheter has a distal and proximal end and parts of the catheter including the first lumen, second lumen, flow distribution region, longitudinal central region, partitioning wall, longitudinal tube each have distal and proximal ends that correspond with distal and proximal ends of the catheter. Where the catheter is connected to the distribution hub, the proximal end of the catheter distribution hub refers to the end connected to the distribution hub.

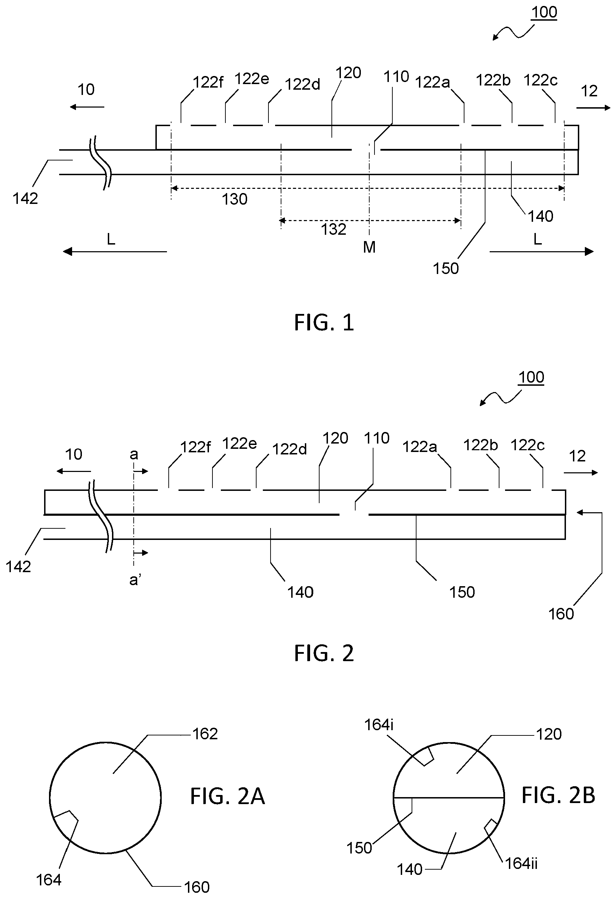

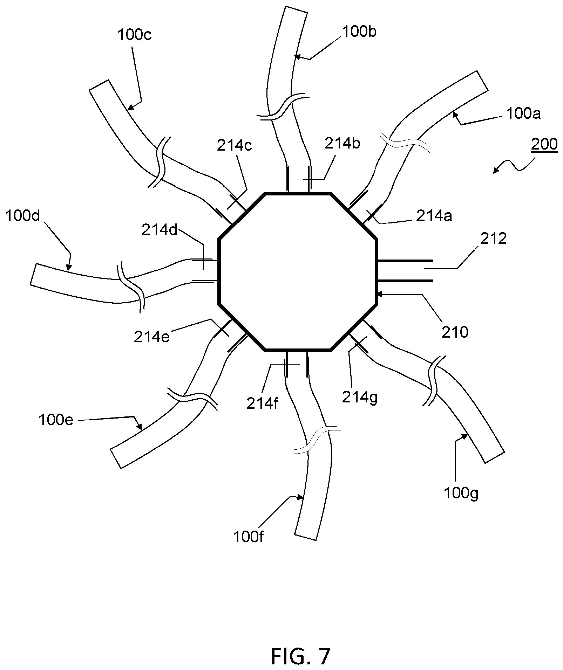

The present invention relates to a catheter for distribution of a liquid outflow. The liquid outflow is distributed at a plurality of outlets along at least a part of a longitudinal length of the catheter. An exemplary catheter (100) is illustrated in **FIGs. 1** to **3****,** and shown also as part of a catheter assembly in **FIG. 7****.**

The catheter (100) has a proximal end (10) and a distal end (12). The catheter (100) comprises a first longitudinal lumen (120) in fluid connection with a plurality of outlets (122a to f) of the first longitudinal lumen (120). The plurality of outlets (122a to f) are disposed in a flow distribution region (130). Each outlet (122a to f) is configured for passage of the liquid outflow.

The catheter (100) further comprises a distribution inlet (110) to the first lumen (120) disposed within a longitudinal central region (132) of the flow distribution region (130). The distribution inlet (110) is configured for passage of the liquid into the first lumen (120).

The catheter (100) further comprises a second longitudinal lumen (140). The second lumen (140) is in fluid connection with the first lumen (120) at the distribution inlet (110). The second lumen (140) is provided at its proximal end (10) with a catheter inlet (142) for inflow of the liquid.

The plurality of outlets (122a to f) is configured to counteract a reduction in outflow as the distance from the distribution inlet (110) increases in the longitudinal direction (L). The outflow may be measured as a quantity (*e.g*. mass, volume) of liquid flowing out from an outlet within a period of time *e.g*. a flow rate.

The inventors have found a reduction in outflow *i.e.* in outflow rate as the distance from the distribution inlet (110) increases in the longitudinal direction (L) when the catheter is not configured according to the present invention. The outflow does not equalise under the pressure used for infusion. The outlets on the extremities (*e.g*. 122c, 122f) of the flow distribution region (130) exhibit a lower outflow rate than those closer to the distribution inlet (110). The unevenness affects distribution within the cavity. The presently invented catheter allows for a more even distribution of outflow along the longitudinal length of the catheter. For intraperitoneal usage, the catheter allows for a more even distribution at different places within the cavity, and avoids that only lower portions of the cavity are treated. It is no longer necessary to fill the cavity in order to treat upper lower portions of the cavity. The patient does not suffer from distention. Smaller volumes of liquid may be used. Continuous infusion may be used which reduces likelihood of resistance (metronomic).

The first longitudinal lumen (120), also known herein as first lumen (120), has a proximal (10) and a distal (12) end. The first longitudinal lumen (120) is closed *i.e.* liquid sealed, except at the distribution inlet (110) and outlets (112a-f)). The first longitudinal lumen (120) is closed (liquid sealed) at its proximal (10) terminal end and/or at its distal (12) terminal end. The first longitudinal lumen (120) may be defined by a longitudinal first inner wall (164i). The longitudinal first inner wall (164i) comprises a part of an inner wall (164) of a tube (160) and one side of a partitioning wall (150) (see later below).

As a general guidance, the first lumen (120) may have a transverse cross-sectional area of, for example, 1.41371 mm² to 1.72788 mm², preferably 1.57079 mm² for intraperitoneal usage. The first lumen (120) may have a length (LL) of, for example, 270 to 330 mm for intraperitoneal usage. The length (LL) of the first lumen (120) is typically measured longitudinally from one closed end (*e.g.* proximal) to the other closed end (*e.g.* distal).

The second longitudinal lumen (140), also known herein as second lumen (140), has a proximal (10) and distal (12) end. The second longitudinal lumen (140) is closed *i.e.* liquid sealed, except at the connection with the distribution inlet (110), and at a catheter inlet (142). The second longitudinal lumen (140) is open at the proximal (10) end; the open proximal end is the main inlet (142). The second longitudinal lumen (140) is closed (liquid sealed) at the distal (12) terminal end. The second longitudinal lumen (140) may be defined by a longitudinal second inner wall (164ii). The longitudinal second inner wall (164ii) comprises a part of an inner wall (164) of a tube (160) and one side of a partitioning wall (150) (see later below).

As a general guidance, the second lumen (140) may have a transverse cross-sectional area of, for example, 1.41371 mm² to 1.72788 mm², preferably 1.57079 mm² for intraperitoneal usage. The second lumen (140) may have a length of, for example, 270 to 330 mm for intraperitoneal usage. The second lumen (140) may have a length less than the first lumen (e.g. less than 270 to 330 mm).

The first (120) and second (140) lumens are at least partly (preferably entirely) parallel. A partitioning wall (150) partitions (fluidly separates) the first (120) and second (140) lumens. The partitioning wall is preferably a single layer, however, it is within the scope of the invention that the partition wall formed from two layers *e.g*. from joining of two walled lumens. The partitioning wall (150) between the first (120) and second (140) lumens fluidly separates them (except at the distribution inlet (110)). The distribution inlet (110) is disposed across the partitioning wall (150).

The first (120) and second (140) lumens are disposed side-by-side. The first (120) and second (140) lumens are non-coaxial. The first (120) and second (140) lumens are non-concentric. The first (120) and second (140) lumens are non-nested *i.e.* not one inside the other.

The first (120) and second (140) lumens may or may not have the same transverse cross-sectional area. The first (120) and second (140) lumens may or may not have the same longitudinal length.

The first (120) and second (140) lumens may have different longitudinal lengths. The second (140) lumen may have a shorter longitudinal length than the first (120) lumen. The first (120) and second (140) lumens may have coinciding proximal terminal ends, and the distal terminal end of the second (140) lumen may be disposed proximal of the distal terminal end of the first lumen (140). Alternatively, the first (120) and second (140) lumens may have coinciding proximal terminal ends, and the distal terminal end of the second (140) lumen may be disposed proximal of the distal terminal end of the first lumen (140) and within the flow distribution region (130), preferably, distal of the distribution inlet (110).

The first (120) and second (140) lumens are comprised in a longitudinal tube (160), also known as a tube (160) herein (**FIG. 2A**). A lumen (162) of the tube (tube lumen (162)) is partitioned (longitudinally) into the first (120) and second (140) lumens by the partitioning wall (150) attached to the tube lumen wall (164). The tube lumen (162) may be segmented (longitudinally) by the partitioning wall (150) (**FIG. 2B**). The catheter (100) may be formed by extrusion process, applied to a partitioned or segmented tubular body. Advantageously, the partitioning wall (150) attached to the tube lumen wall (164) ensures that a longitudinal distance between the distribution inlet (110) and each of the outlets (122a to f) remains constant even when the tube is curved or bent by preventing axial slide.

Advantageously, the partitioning wall (150) attached to the tube lumen wall (164), results in a homogenous outflow of the drug over the length of the catheters, which translates in an improved drug distribution and increased peritoneal surface exposure, the latter enabling improved treatment of peritoneal disease.

As a general guidance, the tube lumen (162) may have a transverse cross-sectional area of, for example, 2.8274 to 3.4558 mm² for intraperitoneal usage.

The catheter (100) may be described as a multi-lumen catheter, and the first (120) and second (140) lumens are lumens of the multi-lumen catheter.

The catheter (100) may be made from any suitable biocompatible material, for instance, metal or alloy (*e.g.* titanium, stainless steel) or polymer (*e.g.* polypropylene, polycarbonate, Teflon).

A longitudinal portion (350) of the partitioning wall (150), a longitudinal portion (350) of the first lumen (120), and a longitudinal portion (350) of the second lumen (140) may be provided in a connecting piece (300) (see below).

At least the flow distribution region (130) may be flexible (*e.g*. for intraperitoneal administration). The tube (160) and partitioning wall (150) may be flexible. At least the flow distribution region (130) may be rigid. The tube (160) and optionally partitioning wall (150) may be rigid.

The flow distribution region (130) is a longitudinal region of the first longitudinal lumen (120). It contains the outlets (122a-f) of the plurality and is limited at either end by the proximal-most (122f) and distal-most (122c) outlets. The outlets (122a-f) are only present in the flow distribution region (130).

The longitudinal central region (132) is a longitudinal subregion of the flow distribution region (130). It preferably has a midpoint (M) that coincides with a midpoint of the longitudinal flow distribution region (130). The longitudinal central region (132) preferably has a length that is no more than 50% of the length of the flow distribution region (130). The distribution inlet (110) is provided within the longitudinal central region (132). The distribution inlet (110) is present only within the longitudinal central region (132). The longitudinal central region (132) may be devoid of outlets (122a-f).

The catheter inlet (142) is disposed at the proximal (10) end of the second longitudinal lumen (140). The catheter inlet (142) may be part of an inlet coupling configured for fluid connection with a complementary coupling, for supply of liquid inflow into the catheter inlet (142). The complementary coupling be a part of a system for inducing a liquid flow (e.g. pump, gravity-fed reservoir).

The distribution inlet is disposed in the partitioning wall (150) separating the first (120) and second lumens (140). In other words, the distribution inlet (110) connects one side of the partitioning wall (150) to the other side. The distribution inlet (110) may have any shape.

Preferably, the distribution inlet (110) is circular, however, other shapes are envisaged including slit, oval, regular polygonal or irregular polygonal shape.

The size of a distribution inlet (110) refers to an area (or derivative such as diameter) of the distribution inlet (110); where the partitioning wall (150) is curved, the area is the curved area.

The distribution inlet (110) is positioned within the longitudinal central region (132) of the flow distribution region (130). Preferably, the distribution inlet (110) is positioned at a midpoint (M) that coincides with a midpoint of the longitudinal flow distribution region (130), and with a midpoint (M) of the longitudinal central region (132)

The quantity of distribution inlets (110) (*i.e.* number count) is preferably only one.

It is within the scope of the present disclosure that the quantity of distribution inlets (110) is two or more. Each and every distribution inlet of the two or more distribution inlets (110) is disposed in the partitioning wall (150) separating the first (120) and second lumens (140). The shape of each and every distribution inlet (110) may be the same (*e.g*. circular, slit, oval, regular polygonal or irregular polygonal shape). The size of each distribution inlet refers to an area (or derivative such as diameter) of the distribution inlet (110); where the partitioning wall (150) is curved, the area is the curved area. Where the quantity of distribution inlets (110) is two or more, each end every distribution inlet (110) is arranged in a different distribution unit. A distribution unit contains the distribution inlet (110) and its own plurality of outlets (122a to f) as described herein. The distribution inlet (110) and plurality of outlets (122a to f) of one distribution unit do not overlap in a longitudinal direction with the distribution inlet and plurality of outlets of another distribution unit. The distribution units are arranged spatially separated from each other in a longitudinal direction.

Each outlet (122a to f) fluidly connects the first lumen (120) to an exterior of the catheter (100).

Each and every outlet (122a to f) of the plurality is separated from the distribution inlet (110) in a longitudinal direction (L). There is preferably no overlap in a longitudinal direction between the distribution inlet (110) and any of the outlets (122a to f). A transverse cross-section of the first longitudinal lumen (120) in a region occupied by the distribution inlet (110) preferably does not contain any part of any outlet (122a to f).

Each and every outlet (122a to f) of the plurality is spatially separated from a neighbouring (adjacent) outlet in the longitudinal (L) direction.

The plurality of outlets (122a to f) is configured such that each provides provide the same outflow rate within a tolerance of ±10 % of a mean outflow rate of the plurality of outlets (122a to f)).

Each and every outlet (122a to f) of the plurality may be formed by laser cutting.

The quantity of outlets (*i.e.* outlet count) may be between 2 and 20, preferably between 4 and 10. The quantity of outlets (122a to c) on the proximal (10) side of the distribution inlet (110) may be equal to the quantity of outlets (122d to f) on the distal (12) side of the distribution inlet (110) ±0, 1, or 2. The quantity of outlets is preferably an even number.

The quantity of outlets (122a to c) on the distal (12) side of the distribution inlet (110) is preferably equal to the quantity of outlets (122d to f) on the proximal (10) side of the distribution inlet (110)

The quantity of outlets (122a to c) on the distal (12) side of the distribution inlet (110) is preferably 3 to 5; the quantity of outlets (122d to f) on the proximal (10) side of the distribution inlet (110) is preferably 3 to 5.

The outlet (122a to f) of the plurality are arranged distributed within the flow distribution region (130). Preferably, each and every outlet (122a to f) of the plurality is separated from the distribution inlet (110) in a longitudinal direction (L).

The outlets (122a to c) of the plurality on the distal (12) side of the distribution inlet (110) are longitudinally positioned and sized in mirrored symmetry with the plurality of outlets (122d to f) on the proximal (10) side of the distribution inlet (110).

The outlets (122a to c) of the plurality on the distal (12) side of the distribution inlet (110) may be unevenly or evenly spaced in a longitudinal direction (L). The outlets (122d to f) of the plurality on the proximal (10) side of the distribution inlet (110) may be unevenly or evenly spaced in a longitudinal direction (L).

The distance between neighbouring pairs of outlets (122d-e, 122e-f) of the plurality on the proximal (10) side of the distribution inlet (110) may decrease as function of longitudinal distance (of the neighbouring pairs) from the distribution inlet (110).

The distance between neighbouring pairs of outlets (122a-b, 122b-c) of the plurality on the distal (12) side of the distribution inlet (110) may decrease as function of longitudinal distance (of the neighbouring pairs) from the distribution inlet (110).

The distance between a neighbouring pair of outlets (e.g. 122a-b, 122b-c 122d-e, 122e-f) is measured as a longitudinal distance between a centre of gravity of a shape of one outlet (*e.g*. circular centre when the outlet is circular) and a centre of gravity of a shape of an adjacent outlet (*e.g*. circular centre when the outlet is circular).

The outlets (122a to c) of the plurality on the distal (12) side of the distribution inlet (110) may be aligned in a longitudinal direction (L) with a straight line, or may not be aligned in a longitudinal direction (L) with a straight line. The outlets (122d to f) of the plurality on the proximal (10) side of the distribution inlet (110) may be aligned in a longitudinal direction (L) with a straight line, or may not be aligned in a longitudinal direction (L) with a straight line.

The size of each and every outlet (122a to c) of the plurality on the distal (12) side of the distribution inlet (110) is different; preferably the size increases as function of longitudinal distance from the distribution inlet (110). In addition, the size of each and every outlet (122d to f) of the plurality on the proximal (10) side of the distribution inlet (110) is different; preferably the size increases as function of longitudinal distance from the distribution inlet (110).

The size of an outlet (122a to f) refers to an area (or derivative such as diameter) of the outlet (122a to f); where the wall of the first lumen (120) is curved, the area is the curved area.

The outlets (122a to f) may have any shape. Preferably, the outlets (122a to f) are circular, but other shapes are envisaged including slit (longitudinal), oval (longitudinal), oblong (square or rounded corners) (longitudinal), regular polygonal or irregular polygonal shape. The shape of each and every outlet (122a to f) may be the same (e.g. circular, slit, oval, oblong, regular polygonal or irregular polygonal). The shape of pairs of outlets having an equal distance from the distribution inlet (110) may be the same. The shape of each and every outlet (122a to f) may be different.

According to one configuration, the distal-most outlet (122c) and the proximal-most outlet (122f) of a flow distribution region (130) have a longitudinal shape, preferably a slit or oblong shape with rounded corners. The other outlets of the flow distribution region (130) may have a circular or longitudinal shape.

According to another configuration,
- the outlet (122d) on the proximal side (10) of the distribution inlet (110) and closest to the distribution inlet (110) is circular,
- the outlet (122a) on the distal side (12) of the distribution inlet (110) and closest to the distribution inlet (110) is circular,
- the other outlets (122b, 122c, 122e, 122f,) are longitudinal, wherein a longitudinal length (L) of each outlet increases as a function of distance of the outlet from the distribution inlet (110).

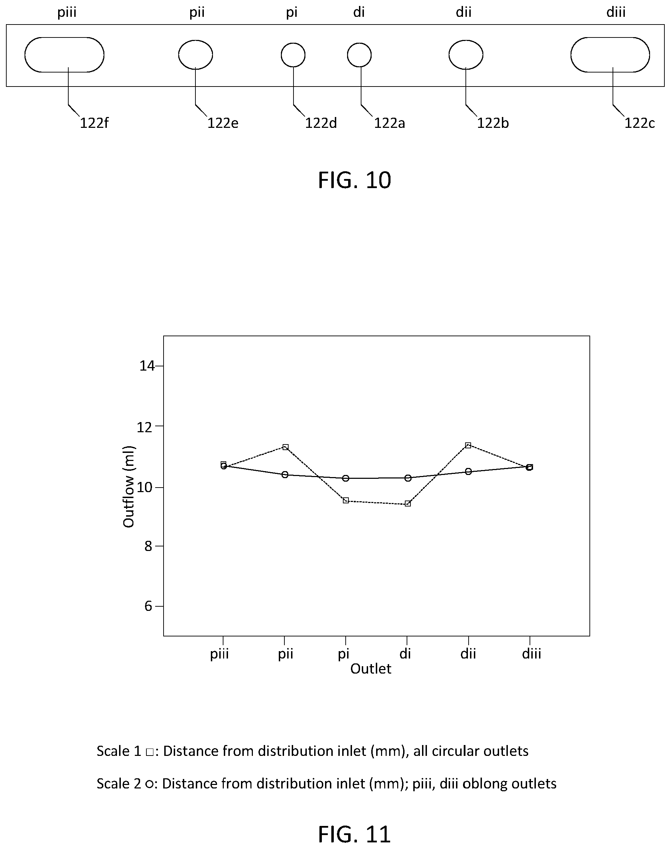

The inventors found that when the distal-most outlet (122c) and the proximal-most outlet (122f) of a flow distribution region (130) have a longitudinal shape, preferably a slit or oblong shape, there is a further improvement in evenness in the outflow over the entire catheter (see **FIG. 11**).

In order to enhance the effect in terms of more homogeneous distributions it is not preferable when all outlets would have a longitudinal shape in the current catheter design with for example six perforations. More specifically, the beneficial effect of a longitudinal shaped outlet with respect to a more homogeneous distribution is expected to be largest for the distal-most outlet (122c) and the proximal-most outlet (122f), as the flow going through these outlets experiences the highest flow resistance. The inventors have found that by enlarging the length of the perforation to obtain a slit and/or oblong shape, outflow resistance can be sensibly reduced.

Advantageously, enlarging the length of the perforation to obtain a slit and/or oblong shape is of particular interest when it is no longer possible to enlarge the diameter of a circular perforation, due to its size relative to the catheter lumen diameter.

Considering the continuity and different orders of momentum for flow in the catheter, the inventors have found that the position and size of each and every outlet (122a to f) determined using the Gaussian quadrature rule gives rise to an even distribution of outflow along the longitudinal length of the flow distribution region.

In Gaussian quadrature, values are generated for a Gauss point set, wherein for each point in the set, a position value and a corresponding weight value is generated. The values are generated according to the known n-point Gaussian quadrature rule of Eq. 1. ${\int }_{a}^{b} f \left(t\right) dt ≅ \frac{b - a}{2} {\sum }_{i = 1}^{n} {w}_{i} f \left(\frac{b - a}{2}{x}_{i}+\frac{b + a}{2}\right)$ which is defined on an interval [a, b] and on the set of distinct points {*x₁, x₂, ..., xₙ*}; wherein:
*t* is an integral variable (position of a point on the interval [a b]);
*n* is quantity of points (Gauss points);
*xᵢ* is position of point *i* on the interval [-1 1] (Gauss position);
*wᵢ* is weight at position *xᵢ* (Gauss weight)

Gauss tables are known in the art listing a position (between -1 and +1) and the corresponding weight (0< *wᵢ ≤* 1 for *n* ≥ *2* and ${\sum }_{i = 1}^{n} {w}_{i} = 2$ with *n= 1, 2,* ...) according to the Gaussian quadrature rule, wherein the positions and weights are provided for a given Gauss point set (*e.g*. 4, 6, 8, 10, 12 or more points). Values corresponding to the Gaussian quadrature rule are set out in **Table 1** below.

**Table 1. Gauss Tables, each Table listing Gauss weights and Gauss positions for a Gauss point sets having 4, 6, 8, 10, or 12 Gauss points for a Gaussian quadrature rule that is Gauss-Legendre quadrature.**

| ***Gauss points*** | ***i*** | **Gauss weight** (*wᵢ*) | **Gauss Position** (*xᵢ*) |
|---|---|---|---|
| 4 | 1 | 0.652145154862 | -0.339981043584 |
| | 2 | 0.652145154862 | 0.339981043584 |
| | 3 | 0.347854845137 | -0.861136311594 |
| | 4 | 0.347854845137 | 0.861136311594 |
| 6 | 1 | 0.360761573048 | 0.661209386466 |
| | 2 | 0.360761573048 | -0.661209386466 |
| | 3 | 0.467913934572 | -0.238619186083 |
| | 4 | 0.467913934572 | 0.238619186083 |
| | 5 | 0.171324492379 | -0.932469514203 |
| | 6 | 0.171324492379 | 0.932469514203 |
| 8 | 1 | 0.362683783378 | -0.183434642495 |
| | 2 | 0.362683783378 | 0.183434642495 |
| | 3 | 0.313706645877 | -0.525532409916 |
| | 4 | 0.313706645877 | 0.525532409916 |
| | 5 | 0.222381034453 | -0.796666477413 |
| | 6 | 0.222381034453 | 0.796666477413 |
| | 7 | 0.101228536290 | -0.960289856497 |
| | 8 | 0.101228536290 | 0.960289856497 |
| 10 | 1 | 0.295524224714 | -0.148874338981 |
| | 2 | 0.295524224714 | 0.148874338981 |
| | 3 | 0.269266719309 | -0.433395394129 |
| | 4 | 0.269266719309 | 0.433395394129 |
| | 5 | 0.219086362515 | -0.679409568299 |
| | 6 | 0.219086362515 | 0.679409568299 |
| | 7 | 0.149451349150 | -0.865063366688 |
| | 8 | 0.149451349150 | 0.865063366688 |
| | 9 | 0.066671344308 | -0.973906528517 |
| | 10 | 0.066671344308 | 0.973906528517 |
| 12 | 1 | 0.249147045813 | -0.125233408511 |
| | 2 | 0.249147045813 | 0.125233408511 |
| | 3 | 0.233492536538 | -0.367831498998 |
| | 4 | 0.233492536538 | 0.367831498998 |
| | 5 | 0.203167426723 | -0.587317954286 |
| | 6 | 0.203167426723 | 0.587317954286 |
| | 7 | 0.160078328543 | -0.769902674194 |
| | 8 | 0.160078328543 | 0.769902674194 |
| | 9 | 0.106939325995 | -0.904117256370 |
| | 10 | 0.106939325995 | 0.904117256370 |
| | 11 | 0.047175336386 | -0.981560634246 |
| | 12 | 0.047175336386 | 0.981560634246 |

The inventors have found that when the number of Gauss points of a Gauss point set corresponds to the quantity of outlets in the first lumen, the Gauss weights correspond to the size of the outlets and the Gauss positions correspond to the locations of the outlets. Following the Gaussian quadrature rule gives an even distribution of outflow along the longitudinal length of the flow distribution region.

The positions of the outlets may be determined from the Gauss position scaled up according to the length (LL) of the first lumen (*e.g*. 300mm). For a chosen Gauss point set (*e.g.* 4, 8, or 10 points), the catheter outlet position from one end of the first lumen may be determined according to the equation LL/2 - (LL/2 **xᵢ*), where LL is the longitudinal length of the first lumen, and *xᵢ* is the Gauss position at point *i* for according to the Gauss point set (of Table 1). The position of an outlet may in addition or alternatively be expressed as a longitudinal distance between the outlet (122a-f) and distribution inlet (110); in such case an equation for calculation an outlet position may be (LL/2 * *xᵢ*), where LL is the longitudinal length of the first lumen, and *xᵢ* is the Gauss position at point *i* for according to the Gauss point set (of Table 1).

The size of the outlets may be determined from the Gauss weights scaled according to the size of the smallest outlet (DD) (*e.g*. 3mm diameter). For a chosen Gauss point set, the size of the catheter outlet at the corresponding position may be determined according to the equation DD * (*wᵢ*/*wₘᵢₙ*), wherein DD is the smallest diameter chosen (*e.g.* by the manufacturer), and *wᵢ* is the Gauss weight at point *i* for according to the Gauss point set, and *wₘᵢₙ* is the smallest Gauss weight within the chosen Gauss point set (of Table 1).

As an example, to determine the positions and sizes of outlets O₋₃, O₋₂, O₋₁, O₊₁, O₊₂, O₊₃, of a six outlet catheter disposed in the written order (O₋₃, O₋₂, O₋₁, O₊₁, O₊₂, O₊₃) from one end (*e.g*. proximal) of the first lumen to the other end (*e.g*. distal), wherein the distribution inlet is located longitudinally midway between O₋₁ and O₊₁, the length (LL) of the first lumen is 300mm, and the diameter of the smallest outlet is 3mm, following procedure may be observed.

To calculate the outlet positions, the Gauss six point set is chosen from **Table 1,** and each Gauss position is scaled up according to the length (LL) of the first lumen, using the formula LL/2 - (LL/2 **xᵢ*). The calculation is set out per outlet in **Table 2:**

**Table 2: Calculation example for positions of six outlets of a catheter using the six point Gauss set of Table 1.**

| **Gauss point (*i*)** | **Gauss Position (*xᵢ*)** | **Catheter outlet** | **Scaling Calculation (LL=300 mm)** | **Catheter outlet position from one end of first lumen** |
|---|---|---|---|---|
| 1 | 0.6612 | O₊₂ | LL/2 - (LL/2 * 0.6612) | 50.82 mm |
| 2 | -0.6612 | O₋₂, | LL/2 - (LL/2 * -0.6612) | 249.18 mm |
| 3 | -0.2386 | O₋₁ | LL/2 - (LL/2 * -0.2386) | 185.79 mm |
| 4 | 0.2386 | O₊₁ | LL/2 - (LL/2 * 0.2386) | 114.21 mm |
| 5 | -0.9325 | O₋₃ | LL/2 - (LL/2 *-0.9325) | 289.87 mm |
| 6 | 0.9325 | O₊₃ | LL/2 - (LL/2 *0.9325) | 10.13 mm |

To calculate the outlet sizes for each corresponding position, the same Gauss six point set is chosen from **Table 1,** and each Gauss weight is scaled up according to the size of the smallest outlet using the formula DD * (*wᵢ* / *wₘᵢₙ*)*.* The calculation is set out per outlet in **Table 3:**

**Table 3: Calculation example for sizes of six outlets of a catheter using the six point Gauss set of Table 1.**

| **Gauss point** | **Gauss weight** (*wᵢ*) | **Catheter outlet** | **Scaling Calculation (DD=3 mm)** | **Diameter catheter outlet** |
|---|---|---|---|---|
| 1 | 0.3607616 | O₊₂ | DD * 0.3607616 / *wₘᵢₙ* | 0.632 mm |
| 2 | 0.3607616 | O₋₂, | DD * 0.3607616 / *wₘᵢₙ* | 0.632 mm |
| 3 | 0.4679139 | O₋₁ | DD * 0.4679139 / *wₘᵢₙ* | 0.819 mm |
| 4 | 0.4679139 | O₊₁ | DD * 0.4679139 / *wₘᵢₙ* | 0.819 mm |
| 5 | 0.1713245 (*wₘᵢₙ*) | O₋₃ | DD * 0.1713245 / *wₘᵢₙ* | 0.3 mm |
| 6 | 0.1713245 (*wₘᵢₙ*) | O₊₃ | DD * 0.1713245 / *wₘᵢₙ* | 0.3 mm |

The position of an outlet may be expressed as a longitudinal distance between the outlet (122a-f) and one end of the first lumen, as exemplified above. It is appreciated that the position of an outlet may in addition or alternatively be expressed as a longitudinal distance between the outlet (122a-f) and distribution inlet (110). In such case equation for calculation an outlet position may be (LL/2 * *xᵢ*). The distance between the outlet (122a-f) and the distribution inlet (110) is measured as a longitudinal distance between a centre of gravity of a shape of the outlet (*e.g*. circular centre when the outlet is circular) and a centre of gravity of a shape of the distribution inlet (110) (*e.g*. circular centre when the outlet is circular).

Factors such as transverse cross-sectional area of the first lumen, temperature, viscosity of the liquid and liquid flow rate have a minimal effect on the position and size of the outlets determined according to Gaussian quadrature.

The plurality of outlets (122a to f) is configured to counteract the reduction in outflow as the distance from the distribution inlet (110) increases in the longitudinal direction (L). The plurality of outlets (122a to f) may be so configured using a plurality of features separately or in combination as already mentioned herein, for instance:
- placing the distribution inlet (110) within a longitudinal central region (132) of the flow distribution region (130),
- the size of each and every outlet (122a to c) of the plurality on the distal (12) side of the distribution inlet (110) is different; preferably the size increases as function of longitudinal distance from the distribution inlet (110),
- the size of each and every outlet (122d to f) of the plurality on the proximal (10) side of the distribution inlet (110) is different; preferably the size increases as function of longitudinal distance from the distribution inlet (110).

The size of each and every outlet (122a to c) as a function of distance from the distribution inlet (110) may be determined according to Equation 1.

The catheters (100) may be disposed with at least one fixation element, configured to co-operate with a suture for suturing the catheter (100) to a structure (*e.g*. tissue) within the body.

The fixation element allows the catheters (100) to be suspended in a (*e.g*. peritoneal) cavity and arranged to reach parts of the cavity *e.g*. extremities of the cavity. The suspended catheters (100) counteract the forces of gravity so that when the subject is standing, the catheters (100) remain in position, and the liquid is still able to contact upper and side reaches of the cavity. The quantity of liquid administered can be reduced, because the liquid outflow is able to contact upper and side reaches of the cavity without having to first fill it.

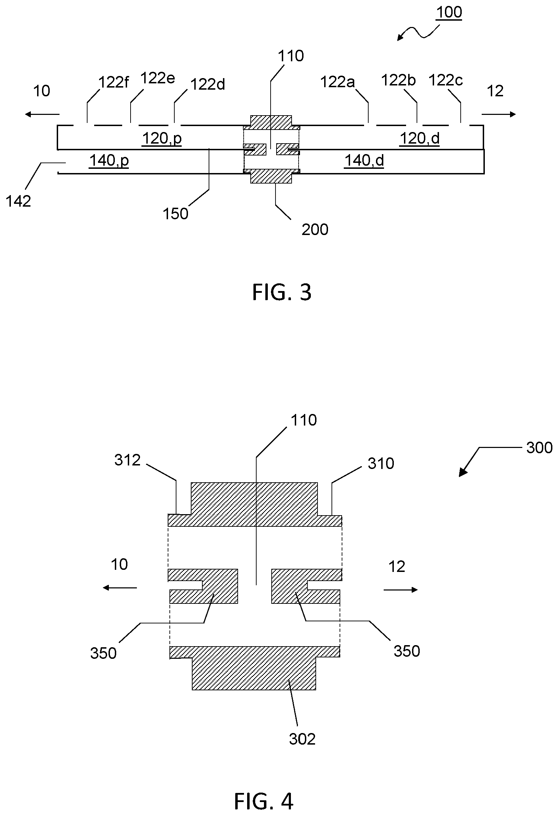

The distribution inlet (110) may be disposed on a connecting piece (300) that is configured to fluidly connect together a proximal portion (120,p) of the first longitudinal lumen (120) to a distal portion (120,d) of the first longitudinal lumen (120), and that fluidly connects together a proximal portion (140,p) of the second longitudinal lumen (140) to a distal portion (140,d) of the second longitudinal lumen (140). An example of the catheter (100) containing the connecting piece (300) is shown in **FIG. 3****.** An example of the connecting piece (300) is shown in **FIG. 4****.**

The connecting piece (300) comprises a body (302) having:
- a proximal (10) end coupling (312) for connection to the proximal portion (120,p) of the first longitudinal lumen (120), and for connection to the proximal portion (140,p) of the second longitudinal lumen (140), and
- a distal (12) end coupling (310) for connection to the distal portion (120,d) of the first longitudinal lumen (120), and for connection to the distal portion (140,d) of the second longitudinal lumen (140), and

The connection is a fluid connection and a mechanical connection. The mechanical connection may be realised by any bonding processes such as adhesive, heat-activated bonding, heat welding.

The connecting piece (300) is configured to fluidly isolate the first and second longitudinal lumens (120, 140) from each other (and from the exterior) except through the distribution inlet (110) disposed on the connecting piece (300).

The partitioning wall (150) between the first and second lumens continues in the connecting piece (300). The connecting piece (300) contains a partitioning wall (350). The distribution inlet (110) is disposed across the partitioning wall (350). The connecting piece (300) partitioning wall (350) fluidly isolates the first and second longitudinal lumens (120, 140) from each other within the connecting piece except through the distribution inlet (110) disposed on the connecting piece (300).

The connecting piece (300) may be made from any suitable biocompatible material, for instance, metal or alloy (*e.g.* titanium, stainless steel) or polymer (*e.g.* polypropylene, polycarbonate, Teflon). The material is preferably lightweight.

The connecting piece (300) assists with manufacture of the catheter (100) by placing the distribution inlet (110) on the connecting piece (300). Introducing a distribution inlet (110) between both two lumens (120, 140) is technically challenging because of the small size and lengths of the lumens. With the connecting piece (300), the distribution inlet (110) can be introduced, for instance, during a moulding process or process to remove a part of the connecting piece (300) partitioning wall (350). The outlets can be readily introduced, e.g. by applying laser cutting to an exterior surface of the wall of the first lumen (120).

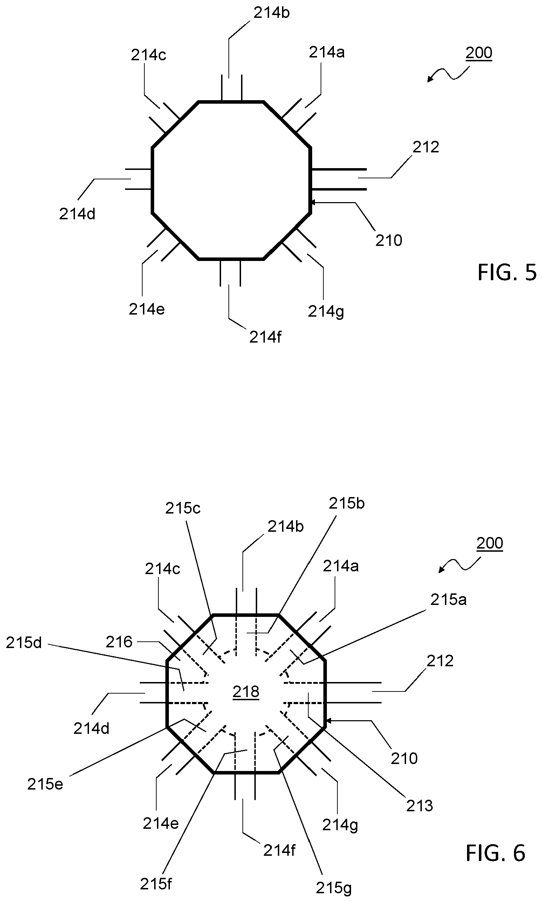

A distribution hub (210) is provided herein. The distribution hub (210) has a hub inlet (212) for inlet of liquid flow, and a plurality of hub outlets (214a to f) for a plurality of liquid outflows. The distribution hub (210) is configured to split the inlet liquid flow into the plurality of liquid outflows. Examples of a distribution hub (210) are shown in **FIGs. 5, 6****,** **7** and **12****.**

The hub outlets (214a to f) all preferably have different directions. The hub outlets (214a to f) all preferably direct liquid outflow in different directions. The hub outlets (214a to f) are preferably arranged at different positions on the distribution hub (210). The hub outlets (214a to f) may have directions that converge at a common point on or within the distribution hub (210). The hub outlets (214a to f) are preferably disposed in a gap on the distribution hub (210) between two planes; the planes may be parallel. Distances between adjacent hub outlets (214a to f) are preferably the same.

Distribution hub may comprise a body (216) containing a chamber (218) for holding the liquid and containing a plurality conduits (215a to f). The body (216) is preferably flat. Each and every conduit (215a to f) may be fluidly connected at one end to the chamber (218). Each and every conduit (215a to f) may be fluidly connected at its other end to its respective hub outlet (214a to f). The quantity of hub outlets (214a to f) may be between 2 and 20, preferably between 5 and 10.

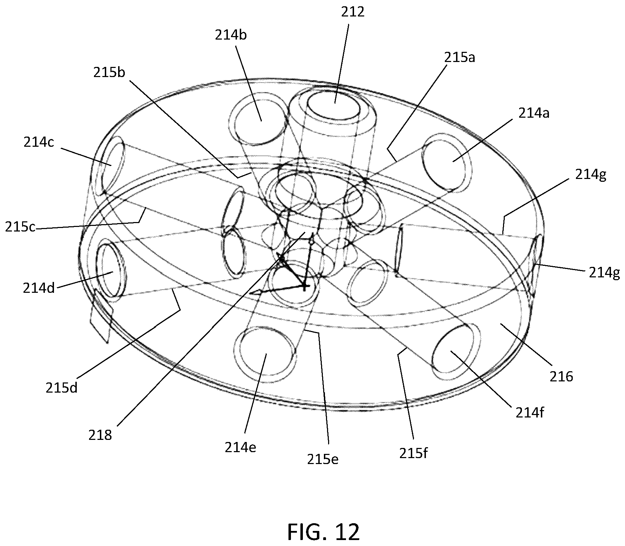

The hub inlet (212) may be fluidly connected to the chamber (218) via an inlet conduit (213). The quantity of hub inlets (212) may be between 1 and 3, preferably only 1. In **FIGs. 5, 6****,** **7****,** the hub inlet (212) and hub outlets (214a to f) have different radial directions that converge at a common point on or within the distribution hub (210). **FIG. 12****,** the hub outlets (214a to f) lie in a common central plane have different radial directions that converge at a common point on or within the distribution hub (210), and the hub inlet (212) is tangential to the common central plane.

Each and every conduit hub outlet (214a to f) of the plurality is configured for fluid connection to a catheter (100a to 100f), in particular to the catheter inlet (142) thereof.

The distribution hub (210) may be made at least partly from any suitable biocompatible material, for instance, metal or alloy (*e.g.* titanium, stainless steel) or polymer (*e.g.* polypropylene, polycarbonate, Teflon).

The distribution hub (210) may be disposed with at least one fixation element, configured to co-operate with a suture for suturing the distribution hub (210) to a structure (e.g. tissue) within the body. The fixation element allows the distribution hub (210) to be suspended in a cavity (*e.g*. centrally) so that the catheters can be arranged to reach extremities of the cavity. The suspended distribution hub (210) counteracts the forces of gravity so that when the subject is standing, the distribution hub (210) remains in position, and the liquid is still able to contact upper and side reaches of the cavity.

The distribution hub increases distribution of the liquid in multiple directions, while having only one inlet to the distribution hub. For cavity (e.g. intraperitoneal applications), the quantity of liquid administered can be reduced, because the liquid outflow is able to contact upper and side reaches of the (e.g. peritoneal) cavity without having to first fill the cavity.

A catheter assembly (200) is provided herein comprising:
- distribution hub (210) as described herein, and
- a plurality of catheters (100a to 100f) as described herein, each operatively connected to a separate hub outlet (214a to f).

Each catheter (100) distribution inlet (110) may be connected to a separate hub outlet (214a to f), via its catheter inlet (142).

Each and every catheter (100a to 100f) of the plurality may be flexible.

At least one, preferably all of the plurality of catheters (100) may each disposed with at least one fixation element, configured to co-operate with a suture for suturing the catheters (100).

The liquid inflow into the catheter inlet (142) is typically under pressure, such that liquid can flow in a proximal (10) to distal (12) direction. Pressure may be generated by a flow inducer such as a pump (e.g. peristaltic pump, or a reciprocating pump) or a gravity feed.

The flow inducer may induce a flow of therapeutic liquid formulation at a rate of 500 to 2000 ml/day for intraperitoneal administration.

The liquid is preferably a therapeutic liquid formulation. The therapeutic liquid formulation preferably contains one or more chemotherapy agents.

The catheter is an infusion catheter. The catheter is an infusion catheter for a cavity. The cavity is a natural cavity and is a peritoneal cavity. It is appreciated, but not currently claimed, that the catheter may be used in other cavities such as for example a pleural cavity (chest), or other cavities created after surgical tumor removal (e.g. retroperitoneal, liver, brain, etc.). The cavity, according to an unclaimed embodiment, may be an artificially generated cavity, such as a surgical generated after removal of tumorous tissue (e.g retroperitoneal, liver, brain, etc.). A vessel for conducing flow of a liquids, such as a blood vessels, lymph vessels, ureter and the like are typically not considered a cavity.

The catheter (100) is an implantable catheter. By implantable catheter, it is meant that at least the flow distribution region (130) is implantable. The whole of the catheter may be implantable.

The catheter (100) may be disposed with an outer sheath. The outer sheath may be protective. The exterior sheath may provide resistance to outward expansion of the first and/or second lumen. The catheter (100) and/or catheter assembly may be disposed with an antibacterial coating (*e.g*. silver).

The catheter may or may not be disposed with one or more additional lumens. An additional lumen may be useful for instance, for taking a sample, air inflation, measurement of pressure, etc.

A system is also provided comprising:
- catheter assembly (200) as described herein or catheter (100) as described herein,
- a reservoir containing therapeutic liquid formulation, and
- a flow inducer to induce a flow of therapeutic liquid formulation from the reservoir to the catheter assembly (200) or catheter (100).

The flow inducer may induce a flow of therapeutic liquid formulation from the reservoir to the catheter assembly (200) or catheter (100) at a rate of 500 to 2000 ml/day for intraperitoneal administration.

Further provided is the presently-described catheter or catheter assembly for use in metronomic delivery of a therapeutic liquid formulation to an intraperitoneal cavity of a subject.

The presently-described catheter or catheter assembly may be used in a method for metronomic delivery of a therapeutic liquid formulation to an intraperitoneal cavity of a subject. The method may comprise the steps:
- implanting a catheter assembly (200) as described herein into an intraperitoneal cavity of the subject, such that at least one of the plurality of catheters (100) is disposed (affixed) in an upper portion of the intraperitoneal cavity,
- after implantation, administering the therapeutic liquid formulation *via* the hub inlet (212), under metronomic conditions (low flow rate, low concentration).

The liquid formulation may contain one or more anti-cancer agents.

According to the presently claimed invention:
- the catheter (100) is for intraperitoneal drug delivery;
- the first (120) and second (140) lumens are comprised in a tube (160) wherein a lumen (162) of the tube, the tube lumen (162), is partitioned into the first (120) and second (140) lumens by a partitioning wall (150) attached to the tube lumen wall (164);
- the outlets (122a to c) of the plurality on the distal (12) side of the distribution inlet (110) are longitudinally positioned and sized in mirrored symmetry with the plurality of outlets (122d to f) on the proximal (10) side of the distribution inlet (110);
- the distal (12) terminal end and proximal (10) terminal end of the first (120) lumen are liquid sealed closed;
- the proximal (10) terminal end of the second (140) lumen is open and forms the catheter inlet (142) for inflow of the liquid.

According to one example:
- the catheter (100) is for intraperitoneal drug delivery;
- the catheter (100) has only 6 outlets (122a-f) and only 1 distribution inlet (110), wherein 3 outlets (122a-c) are disposed on the distal (12) side of the distribution inlet (110) and 3 outlets (122d-f) are disposed on the proximal (10) side of the distribution inlet (110);
- the first (120) and second (140) lumens are comprised in a tube (160) wherein a lumen (162) of the tube (tube lumen (162)) has a transverse cross-sectional area of 2.827 mm² to 3.456 mm² preferably 3.142 mm², and is partitioned preferably equally into the first (120) and second (140) lumens by a partitioning wall (150) attached to the tube lumen wall (164);
- the length of the first (120) lumen is 270 to 300 mm, preferably 300 mm;
- the distribution inlet (110) has an area of 1.8 to 2.2 mm², preferably 2 mm²;
- areas of the 3 outlets (122a-c) disposed on the distal (12) side of the distribution inlet (110) are i) 0.0777 mm² to 0.0636 mm² preferably 0.0707 mm² (122a), ii) 0.143 mm² to 0.175 mm² preferably 0.159 mm² (122b), iii) 0.475 mm² to 0.580613 mm² preferably 0.52783 mm² (122c) in sequence in a proximal (10) to distal (12) direction;
- areas of the 3 outlets (122d-f) disposed on the proximal (10) side of the distribution inlet (110) are i) 0.0777 mm² to 0.0636 mm² preferably 0.0707 mm² (122d), ii) 0.1431 mm² to 0.175 mm² preferably 0.159 mm² (122e), iii) 0.475 mm² to 0.580613 mm² preferably 0.52783 mm² (122f) in sequence in a distal (12) to proximal (10) direction;
- distances of the 3 outlets (122a-c) disposed on the distal side (12) of the distribution inlet (110) to the distribution inlet (110) are i) 35.211 mm to 36.369 mm preferably 35.79 mm (122a), ii) 89.182 mm to 109.098 mm preferably 99.18 mm (122b), iii) 125.883 mm to 153.857 mm preferably 139.87 mm (122c) in sequence in a proximal (10) to distal (12) direction;
- distances of the 3 outlets (122d-f) disposed on the proximal (10) side of the distribution inlet (110) to the distribution inlet (110) are i) 35.211 mm to 36.369 mm preferably 35.79 mm (122d), ii) 89.182 mm to 109.098 mm preferably 99.18 mm (122e), iii) 125.883 mm to 153.857 mm preferably 139.87 mm (122f) in sequence in a distal (12) to proximal (10) direction;
- the outlets (122a-f) are all circular;
- the distal (12) terminal end and proximal (10) terminal end of the first (120) lumen are closed (liquid sealed);
- the distal (12) terminal end of the second (140) lumen is closed (liquid sealed);
- the proximal (10) terminal end of the second (140) lumen is open and forms the catheter inlet (142) for inflow of the liquid.

### Experimental example 1

A catheter was prepared according to the disclosure herein, having the following characteristics:
- 6 outlets and 1 distribution inlet wherein 3 outlets were disposed on the distal side of the distribution inlet and 3 outlets were disposed on the proximal side of the distribution inlet;
- the first and second lumens were comprised in a tube wherein the tube lumen had a transverse cross-sectional area of 3.14 mm², and is partitioned equally into the first (120) and second lumens by a partitioning wall attached to the tube lumen wall;
- the length of the first lumen was 300 mm;
- the length of the second lumen was 300 mm;
- the distribution inlet had an area of 2 mm²;
- areas of the 3 outlets disposed on the distal side of the distribution inlet were di) 0.0707 mm², dii) 0.159 mm², diii) 0.528 mm² in sequence in a proximal to distal direction;
- areas of the 3 outlets disposed on the proximal side of the distribution inlet were pi) 0.0707 mm², pii) 0.159 mm², piii) 0.528 mm² in sequence in a distal to proximal direction;
- distances of the 3 outlets disposed on the distal side of the distribution inlet (110) to the distribution inlet were di) 35.79 mm, dii) 99.18 mm, diii) 139.87 mm in sequence in a proximal to distal direction;
- distances of the 3 outlets disposed on the proximal side of the distribution inlet to the distribution inlet were pi) 35.79 mm, pii) 99.18 mm, piii) 139.87 mm in sequence in a distal to proximal direction;
- the shape of each outlet was circular.

The above characteristics were obtained based on Eq. 1 herein. Outlets on the first lumen were created using a laser instrument. The catheter inlet was connected to an infusion pump, and water was pumped through at a flow rate of 71.4 ml/day for one branch. Outflow from each outlet was separately collected for a test duration and the weight was measured. The experiment was repeated three times.

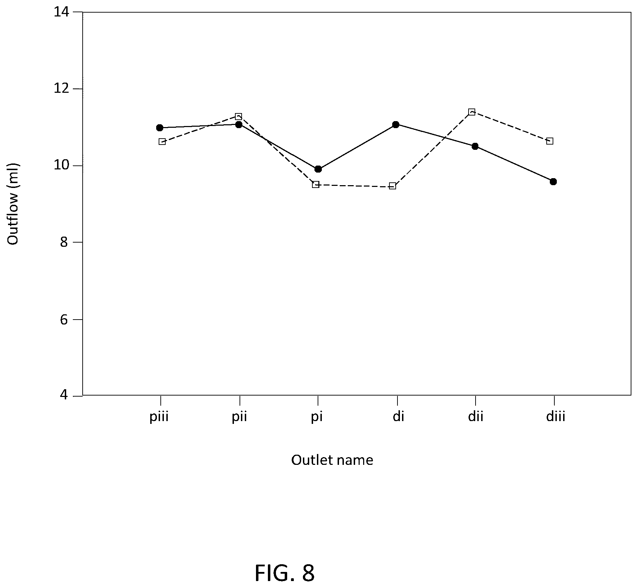

The results shown in **FIG. 8** demonstrate that each outlet produced a similar outflow quantity for the test duration (solid line, solid circles). The experimental data also agrees with a simulation of the flow rates obtained using computational fluid dynamics (CFD) (dashed line, squares) of the same catheter with the same characteristics. For the CFD simulation, a 3D model of perforated catheter was generated using COMSOL Multiphysics (COMSOL, Inc., Burlington, VT,USA). Initial and boundary conditions were assumed for the flow simulation using CFD Module using COMSOL Multiphysics.

### Experimental example 2

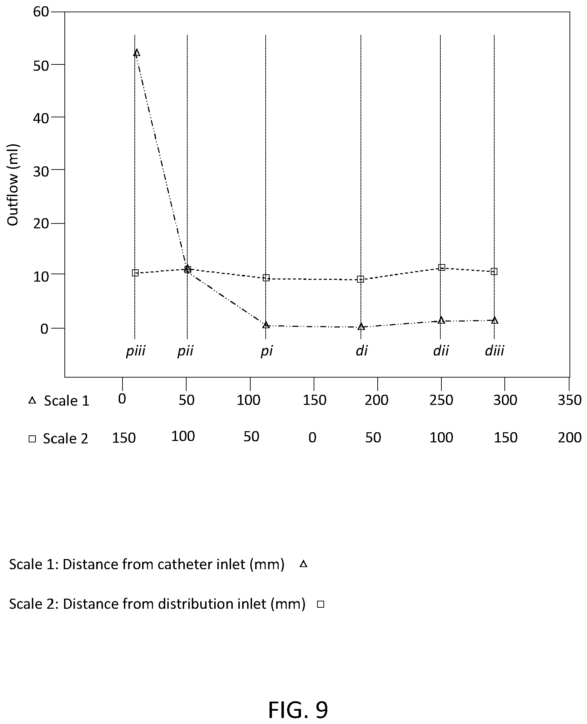

As a further comparison, CFD was performed on a similar catheter, not of the invention, wherein the size of each and every outlet was the same, and the inlet to the first lumen was disposed at the proximal end of the first lumen. This was compared to the CDF performed on a catheter of the invention mentioned above. The results are shown in **FIG. 9****,** where in the catheter not of the invention (dashed-dotted line, triangles) more than 80% of the liquid was lost through outlets pi, pii, and piii, and almost no flow was present through outlets di, dii, and diii. This compares with the CFD simulation of the catheter of the invention (dashed line, squares), having a more even outflow distribution. The results demonstrate a large improvement in flow distribution.

### Experimental example 3

A further CFD simulation of the catheter according to Example 1 was performed in which the shape of outlets piii) and diii) were oblong as exemplarily illustrated in **FIG. 10****.** The results are shown in **FIG. 11****,** wherein the catheter having all circular outlets (dashed line, open squares) is reproduced from FIG. 9, and overlaid is the outflow for a catheter in which the distal-most outlet (diii) and the proximal-most outlet (piii) of the flow distribution region have an oblong shape (solid line, open circles). The results demonstrate a further improvement in flow distribution when oblong-shaped outlets are used for the extremities of the flow distribution region.

## Claims

1. A catheter (100) for intraperitoneal drug delivery having a proximal (10) end and a distal (12) end for distribution of a liquid outflow, the catheter (100) comprising:
- a first longitudinal lumen (120) disposed with a plurality of outlets (122a to f) in a flow distribution region (130) for passage of the liquid outflow,
- a distribution inlet (110) to the first lumen (120) disposed within a longitudinal central region (132) of the flow distribution region (130),
- a second longitudinal lumen (140), wherein the second lumen (140) is in fluid connection with the first lumen (120) at the distribution inlet (110), and is provided at a proximal (10) end with a catheter inlet (142) for inflow of the liquid,
- the first (120) and second (140) lumens are comprised in a tube (160) wherein a lumen (162) of the tube is partitioned into the first (120) and second (140) lumens by a partitioning wall (150) attached to the tube lumen wall (164),
- the outlets (122a to c) of the plurality on the distal (12) side of the distribution inlet (110) are longitudinally positioned and sized in mirrored symmetry with the plurality of outlets (122d to f) on the proximal (10) side of the distribution inlet (110),
- the distal (12) terminal end and proximal (10) terminal end of the first (120) lumen are liquid sealed closed,
- the distal (12) terminal end of the second (140) lumen is liquid sealed closed,
- the proximal (10) terminal end of the second (140) lumen is open and forms the catheter inlet (142) for inflow of the liquid,
wherein the plurality of outlets (122a to f) is configured to counteract a reduction in outflow as the distance from the distribution inlet (110) increases in the longitudinal direction (L).

2. The catheter (100) according to claim 1, wherein the quantity of outlets (122d to f) on the proximal (10) side of the distribution inlet (110) is equal to the quantity of outlets (122a to c) on the distal (12) side of the distribution inlet (110) ±0, 1, or 2.

3. The catheter (100) according to claim 1 or 2, wherein the longitudinal central region (132) of the flow distribution region (130):
- has a length that is no more than 50% of the length of the flow distribution region (130), and
- has a midpoint (M) that coincides with a midpoint of the longitudinal flow distribution region (130).

4. The catheter (100) according to any one of claims 1 to 3, disposed with at least one fixation element, configured to co-operate with a suture for suturing the catheter (100) to a structure within the body.

5. The catheter (100) according to any one of claims 1 to 4, wherein each and every outlet (122a to f) of the plurality is separated from the distribution inlet (110) in a longitudinal direction (L).

6. The catheter (100) according to any one of claims 1 to 5, wherein:
- the size of each and every outlet (122d to f) of the plurality on the proximal (10) side of the distribution inlet (110) is different, and the size increases as function of longitudinal distance from the distribution inlet (110); and
- the size of each and every outlet (122a to c) of the plurality on the distal (12) side of the distribution inlet (110) is different, and the size increases as function of longitudinal distance from the distribution inlet (110).

7. The catheter (100) according to any one of claim 1 to 6, wherein:
- a distance between neighbouring pairs of outlets (122d-e, 122e-f) of the plurality on the proximal (10) side of the distribution inlet (110) decreases as function of longitudinal distance of the neighbouring pairs from the distribution inlet (110); and
- a distance between neighbouring pairs of outlets (122a-b, 122b-c) of the plurality on the distal (12) side of the distribution inlet (110) decreases as function of longitudinal distance of the neighbouring pairs from the distribution inlet (110).

8. The catheter (100) according to any one of claims 1 to 7, wherein position and size of each and every outlet (122a to f) is determined according to a Gaussian quadrature rule, wherein
- the Gaussian quadrature rule defines, for each point of a Gauss point set of *n* Gauss points, a Gauss weight and a Gauss position, and
- the quantity of outlets of the catheter (100) corresponds to *n,* the Gauss positions of the Gauss point set of *n* Gauss points correspond to the positions of the outlets (122a to f), and the Gauss weights of the Gauss point set of *n* Gauss points correspond to the size of the outlets.

9. The catheter (100) according to any one of claims 1 to 8, wherein the catheter is an implantable catheter wherein at least the flow distribution region (130) is implantable.

10. The catheter (100) according to any one of claims 1 to 9, wherein the distribution inlet (110) is disposed on a connecting piece (300), wherein the connecting piece (300) comprises a body (302) having:
- a proximal (10) end coupling (312) for connection to a proximal portion (120,p) of the first longitudinal lumen (120), and for connection to a proximal portion (140,p) of the second longitudinal lumen (140), and
- a distal (12) end coupling (310) for connection to a distal portion (120,d) of the first longitudinal lumen (120), and for connection to a distal portion (140,d) of the second longitudinal lumen (140), and
wherein the connecting piece (300) is configured to:
- fluidly connect:
- the proximal portion (120,p) of the first longitudinal lumen (120) to the distal portion (120,d) of the first longitudinal lumen (120), and
- the proximal portion (140,p) of the second longitudinal lumen (140) to the distal portion (140,d) of the second longitudinal lumen (140),
- fluidly isolate the first and second longitudinal lumens (120, 140) from each other except through the distribution inlet (110) disposed on the connecting piece (300).

11. A catheter assembly (200) comprising:
- a distribution hub (210) having a hub inlet (212) for inlet of liquid flow, and a plurality of hub outlets (214a to f) for a plurality of liquid outflows, where the distribution hub (210) is configured to split the inlet liquid flow into the plurality of liquid outflows,
- a plurality of catheters (100a to 100f) according to any one of claims 1 to 10, each operatively connected to a separate hub outlet (214a to f).

12. The catheter assembly (200) according to claim 11, wherein:
- the distribution hub comprises a body (216) containing a chamber (218) for holding the liquid and containing a plurality conduits (215a to f),
- each and every conduit (215a to f) is fluidly connected at one end to the chamber (218), and at its other end to its respective hub outlet (214a to f),
- the hub inlet (212) is fluidly connected to the chamber (218) via an inlet conduit (213).

13. The catheter assembly (200) according to claim 11 or 12, wherein the distribution hub (210) is disposed with at least one fixation element, configured to co-operate with a suture for suturing the distribution hub (210) to a structure within the body.

14. A system comprising:
- a catheter (100) according to any one of claims 1 to 10, or a catheter assembly (200) according to any one of claims 11 to 13,
- a reservoir containing the liquid that is a therapeutic liquid formulation, and
- a flow inducer configured to induce a flow of therapeutic liquid formulation from the reservoir to the catheter (100) or catheter assembly (200).

## Patentansprüche

1. Katheter (100) zur intraperitonealen Arzneimittelabgabe mit einem proximalen (10) Ende und einem distalen (12) Ende zur Verteilung eines Flüssigkeitsausflusses, wobei der Katheter (100) umfasst:
- ein erstes Längslumen (120), das mit einer Vielzahl von Auslässen (122a bis f) in einem Flussverteilungsbereich (130) zum Durchlass des Flüssigkeitsauslasses angeordnet ist,
- einen Verteilungseinlass (110) in das erste Lumen (120), das innerhalb eines Längsmittelbereichs (132) des Flussverteilungsbereichs (130) angeordnet ist,
- ein zweites Längslumen (140), wobei das zweite Lumen (140) an dem Verteilungseinlass (110) mit dem ersten Lumen (120) in Fluidverbindung ist und an einem proximalen (10) Ende mit einem Kathetereinlass (142) für den Zufluss der Flüssigkeit versehen ist,
- das erste (120) und das zweite (140) Lumen liegen in einem Schlauch (160), wobei ein Lumen (162) des Schlauchs durch eine Trennwand (150), die an der Schlauchlumenwand (164) angebracht ist, in das erste (120) und das zweite (140) Lumen getrennt ist,
- die Auslässe (122a bis c) der Vielzahl auf der distalen (12) Seite des Verteilungseinlasses (110) sind in Längsrichtung positioniert und in spiegelbildlicher Symmetrie mit der Vielzahl von Auslässen (122d bis f) auf der proximalen (10) Seite des Verteilungseinlasses (110) bemessen,
- das distale (12) terminale Ende und das proximale (10) terminale Ende des ersten (120) Lumens sind flüssigkeitsdicht verschlossen,
- das distale (12) terminale Ende des zweiten (140) Lumens ist flüssigkeitsdicht verschlossen,
- das proximale (10) terminale Ende des zweiten (140) Lumens ist offen und bildet den Kathetereinlass (142) für den Zufluss der Flüssigkeit,
wobei die Vielzahl von Auslässen (122a bis f) dazu ausgelegt ist, einer Verringerung im Abfluss entgegenzuwirken, wenn der Abstand vom Verteilungseinlass (110) in der Längsrichtung (L) zunimmt.

2. Katheter (100) nach Anspruch 1, wobei die Menge an Auslässen (122d bis f) auf der proximalen (10) Seite des Verteilungseinlasses (110) gleich der Anzahl an Auslässen (122a bis c) auf der distalen (12) Seite des Verteilungseinlasses (110) ±0, 1 oder 2 ist.

3. Katheter (100) nach Anspruch 1 oder 2, wobei der Längsmittelbereich (132) des Flussverteilungsbereiches (130):
- eine Länge von maximal 50% der Länge des Flussverteilungsbereiches (130) aufweist, und
- einen Mittelpunkt (M) aufweist, der mit einem Mittelpunkt des Flussverteilungsbereiches (130) zusammenfällt.

4. Katheter (100) nach einem der Ansprüche 1 bis 3, der mit mindestens einem Fixierelement versehen ist, das dazu ausgelegt ist, mit einem Nahtfaden zum Nähen des Katheters (100) an eine Struktur innerhalb des Körpers zusammenzuwirken.

5. Katheter (100) nach einem der Ansprüche 1 bis 4, wobei jeder einzelne Auslass (122a bis f) der Vielzahl in einer Längsrichtung (L) vom Verteilungseinlass (110) getrennt ist.

6. Katheter (100) nach einem der Ansprüche 1 bis 5, wobei:
- die Größe eines jeden einzelnen Auslasses (122d bis f) der Vielzahl auf der proximalen (10) Seite des Verteilungseinlasses (110) anders ist, und die Größe als Funktion des Längsabstandes vom Verteilungseinlass (110) zunimmt, und
- die Größe eines jeden einzelnen Auslasses (122a bis c) der Vielzahl auf der distalen (12) Seite des Verteilungseinlasses (110) anders ist, und die Größe als Funktion des Längsabstandes vom Verteilungseinlass (110) zunimmt.

7. Katheter (100) nach einem der Ansprüche 1 bis 6, wobei:
- ein Abstand zwischen benachbarten Paaren von Auslässen (122d-e, 122e-f) der Vielzahl auf der proximalen (10) Seite des Verteilungseinlasses (110) als Funktion des Längsabstandes der benachbarten Paare vom Verteilungseinlass (110) abnimmt; und
- ein Abstand zwischen benachbarten Paaren von Auslässen (122a-b, 122b-c) der Vielzahl auf der distalen (12) Seite des Verteilungseinlasses (110) als Funktion des Längsabstandes der benachbarten Paare vom Verteilungseinlass (110) abnimmt.

8. Katheter (100) nach einem der Ansprüche 1 bis 7, wobei Position und Größe von jedem einzelnen Auslass (122a bis f) gemäß einer Gaußschen Quadraturregel bestimmt werden, wobei
- die Gaußsche Quadraturregel für jeden Punkt einer Gaußschen Punktmenge von n Gaußschen Punkten ein Gaußsches Gewicht und eine Gaußsche Position definiert, und
- die Anzahl an Auslässen des Katheters (100) n entspricht, die Gaußschen Positionen der Gaußschen Punktmenge von n Gaußschen Punkten den Positionen der Auslässe (122a bis f) entsprechen und die Gaußschen Gewichte der Gaußschen Punktmenge von n Gaußschen Punkten der Größe der Auslässe entsprechen.

9. Katheter (100) nach einem der Ansprüche 1 bis 8, wobei der Katheter ein implantierbarer Katheter ist, wobei zumindest der Flussverteilungsbereich (130) implantierbar ist.

10. Katheter (100) nach einem der Ansprüche 1 bis 9, wobei der Verteilungseinlass (110) an einem Verbindungsstück (300) angeordnet ist, wobei das Verbindungsstück (300) einen Körper (302) umfasst mit:
- einer Kopplung (312) am proximalen (10) Ende zur Verbindung mit einem proximalen Abschnitt (120,p) des ersten Längslumens (120) und zur Verbindung mit einem proximalen Abschnitt (140,p) des zweiten Längslumens (140), und
- einer Kopplung (310) am distalen (12) Ende zur Verbindung mit einem distalen Abschnitt (120,d) des ersten Längslumens (120) und zur Verbindung mit einem distalen Abschnitt (140,d) des zweiten Längslumens (140), und
wobei das Verbindungsstück (300) dazu ausgelegt ist:
- fluidisch zu verbinden:
- den proximalen Abschnitt (120,p) des ersten Längslumens (120) mit dem distalen Abschnitt (120,d) des ersten Längslumens (120), und
- den proximalen Abschnitt (140,p) des zweiten Längslumens (140) mit dem distalen Abschnitt (140,d) des zweiten Längslumens (140),
- das erste und das zweite Längslumen (120, 140) fluidisch voneinander zu isolieren, mit Ausnahme des Verteilungseinlasses (110), der an dem Verbindungsstück (300) angeordnet ist.

11. Katheteranordnung (200), umfassend:
- einen Verteilungshub (210) mit einem Hubeinlass (212) für den Einlass eines Flüssigkeitsflusses, und eine Vielzahl von Hubauslässen (214a bis f) für eine Vielzahl von Flüssigkeitsauslässen, wobei der Verteilungshub (210) dazu ausgelegt ist, den Einlassflüssigkeitsfluss auf die Vielzahl von Flüssigkeitsausflüssen aufzuteilen,
- eine Vielzahl von Kathetern (100a bis 100f) nach einem der Ansprüche 1 bis 10, die jeweils funktional mit einem separaten Hubauslass (214a bis f) verbunden sind.

12. Katheteranordnung (200) nach Anspruch 11, wobei:
- der Verteilungshub einen Körper (216) umfasst, der eine Kammer (218) zum Aufnehmen der Flüssigkeit enthält und eine Vielzahl von Leitungen (215a bis f) enthält,
- jede einzelne Leitung (215a bis f) fluidisch mit einem Ende der Kammer (218) verbunden ist und an ihrem anderen Ende mit ihrem entsprechenden Hubauslass (214a bis f) verbunden ist,
- der Hubeinlass (212) über eine Einlassleitung (213) fluidisch mit der Kammer (218) verbunden ist.

13. Katheteranordnung (200) nach Anspruch 11 oder 12, wobei der Verteilungshub (210) mit mindestens einem Fixierelement versehen ist, das dazu ausgelegt ist, mit einem Nahtfaden zum Nähen des Verteilungshubs (210) an eine Struktur innerhalb des Körpers zusammenzuwirken.

14. System, umfassend:
- einen Katheter (100) nach einem der Ansprüche 1 bis 10 oder eine Katheteranordnung (200) nach einem der Ansprüche 11 bis 13,
- ein Reservoir, das die Flüssigkeit enthält, die eine therapeutische Flüssigformulierung ist, und
- einen Flussinduzierer, der dazu ausgelegt ist, einen Fluss von therapeutischer Flüssigformulierung von dem Reservoir zum Katheter (100) oder zur Katheteranordnung (200) zu induzieren.

## Revendications

1. Cathéter (100) pour l'administration intrapéritonéale de médicament ayant une extrémité proximale (10) et une extrémité distale (12) pour la distribution d'un flux sortant de liquide, le cathéter (100) comprenant :
- une première lumière longitudinale (120) disposée avec une pluralité de sorties (122a à f) dans une région de répartition de flux (130) pour le passage du flux sortant de liquide,
- une entrée de distribution (110) vers la première lumière (120) disposée au sein d'une région centrale longitudinale (132) de la région de distribution de flux (130),
- une seconde lumière longitudinale (140), dans lequel la seconde lumière (140) est en liaison fluidique avec la première lumière (120) au niveau de l'entrée de distribution (110), et est pourvue, au niveau d'une extrémité proximale (10), d'une entrée de cathéter (142) pour le flux entrant du liquide,
- les première (120) et seconde (140) lumières sont comprises dans un tube (160) dans lequel une lumière (162) du tube est cloisonnée en première (120) et seconde (140) lumières par une paroi de cloisonnement (150) fixé à la paroi de lumière de tube (164),
- les sorties (122a à c) de la pluralité sur le côté distal (12) de l'entrée de distribution (110) sont positionnées longitudinalement et dimensionnées de manière symétrique avec la pluralité de sorties (122d à f) sur le côté proximal (10) de l'entrée de distribution (110),
- l'extrémité terminale distale (12) et l'extrémité terminale proximale (10) de la première (120) lumière sont étanches au liquide,
- l'extrémité terminale distale (12) de la seconde (140) lumière est étanche au liquide,
- l'extrémité terminale proximale (10) de la seconde (140) lumière est ouverte et forme l'entrée du cathéter (142) pour le flux entrant du liquide,
dans lequel la pluralité de sorties (122a à f) est configurée pour s'opposer à une réduction du flux sortant à mesure que la distance depuis l'entrée de distribution (110) augmente dans la direction longitudinale (L).

2. Cathéter (100) selon la revendication 1, dans lequel la quantité de sorties (122d à f) sur le côté proximal (10) de l'entrée de distribution (110) est égale à la quantité de sorties (122a à c) sur le côté distal (12) de l'entrée de distribution (110) ±0, 1 ou 2.

3. Cathéter (100) selon la revendication 1 ou 2, dans lequel la région centrale longitudinale (132) de la région de distribution de flux (130) :
- présente une longueur qui n'est pas supérieure à 50 % de la longueur de la région de distribution de flux (130), et
- présente un point milieu (M) qui coïncide avec un point milieu de la région de distribution de flux (130) longitudinale.

4. Cathéter (100) selon l'une quelconque des revendications 1 à 3, disposé avec au moins un élément de fixation, configuré pour coopérer avec une suture pour suturer le cathéter (100) à une structure à l'intérieur du corps.

5. Cathéter (100) selon l'une quelconque des revendications 1 à 4, dans lequel chaque sortie (122a à f) de la pluralité est séparée de l'entrée de distribution (110) dans une direction longitudinale (L).

6. Cathéter (100) selon l'une quelconque des revendications 1 à 5, dans lequel
- la dimension de chaque sortie (122d à f) de la pluralité sur le côté proximal (10) de l'entrée de distribution (110) est différente, et la taille augmente en fonction de la distance longitudinale à partir de l'entrée de distribution (110) ; et
- la dimension de chaque sortie (122a à c) de la pluralité sur le côté distal (12) de l'entrée de distribution (110) est différente, et la taille augmente en fonction de la distance longitudinale à partir de l'entrée de distribution (110).

7. Cathéter (100) selon l'une quelconque des revendications 1 à 6, dans lequel
- une distance entre des paires de sorties (122d-e, 122e-f) voisines de la pluralité sur le côté proximal (10) de l'entrée de distribution (110) diminue en fonction de la distance longitudinale des paires voisines à partir de l'entrée de distribution (110) ; et
- une distance entre des paires de sorties (122a-b, 122b-c) voisines de la pluralité sur le côté distal (12) de l'entrée de distribution (110) diminue en fonction de la distance longitudinale des paires voisines à partir de l'entrée de distribution (110).

8. Cathéter (100) selon l'une quelconque des revendications 1 à 7, dans lequel la position et la taille de chaque sortie (122a à f) sont déterminées selon une règle de quadrature gaussienne, dans lequel
- la règle de quadrature gaussienne définit, pour chaque point d'un ensemble de points de Gauss de n points de Gauss, un poids de Gauss et une position de Gauss, et
- la quantité de sorties du cathéter (100) correspond à n, les positions de Gauss de l'ensemble de points de Gauss de n points de Gauss correspondent aux positions des sorties (122a à f), et les poids de Gauss de l'ensemble de points de Gauss de n points de Gauss correspondent à la taille des sorties.

9. Cathéter (100) selon l'une quelconque des revendications 1 à 8, le cathéter étant un cathéter implantable dans lequel au moins la région de distribution de flux (130) est implantable.

10. Cathéter (100) selon l'une quelconque des revendications 1 à 9, dans lequel l'entrée de distribution (110) est disposée sur une pièce de liaison (300), dans lequel la pièce de liaison (300) comprend un corps (302) ayant :
- un accouplement d'extrémité proximal (10) (312) pour une liaison à une partie proximale (120,p) de la première lumière longitudinale (120), et pour une liaison à une partie proximale (140,p) de la seconde lumière longitudinale (140), et
- un accouplement d'extrémité distal (12) (310) pour une liaison à une partie distale (120,d) de la première lumière longitudinale (120), et pour une liaison à une partie distale (140,d) de la seconde lumière longitudinale (140), et
dans lequel la pièce de liaison (300) est configurée pour :
- relier fluidiquement :
- la partie proximale (120,p) de la première lumière longitudinale (120) à la partie distale (120,d) de la première lumière longitudinale (120), et
- la partie proximale (140,p) de la seconde lumière longitudinale (140) à la partie distale (140,d) de la seconde lumière longitudinale (140), et
- isoler fluidiquement les première et seconde lumières longitudinales (120, 140) l'une de l'autre sauf à travers l'entrée de distribution (110) disposée sur la pièce de liaison (300).

11. Ensemble cathéter (200) comprenant :
- une embase de distribution (210) présentant une entrée d'embase (212) pour l'entrée de flux de liquide, et une pluralité de sorties d'embase (214a à f) pour une pluralité de flux sortants de liquide, où l'embase de distribution (210) est configurée pour diviser le flux de liquide d'entrée en la pluralité de flux de liquide,
- une pluralité de cathéters (100a à 100f) selon l'une quelconque des revendications 1 à 10, chacun raccordé fonctionnellement à une sortie d'embase (214a à f) distincte.

12. Ensemble cathéter (200) selon la revendication 11, dans lequel :
- l'embase de distribution comprend un corps (216) contenant une chambre (218) pour renfermer le liquide et contenant une pluralité de conduits (215a à f),
- chaque conduit (215a à f) est relié fluidiquement à la chambre (218) au niveau d'une extrémité, et à sa sortie d'embase (214a à f) respective au niveau de son autre extrémité,
- l'entrée d'embase (212) est reliée fluidiquement à la chambre (218) par le biais d'un conduit d'entrée (213).

13. Ensemble cathéter (200) selon la revendication 11 ou 12, dans lequel l'embase de distribution (210) est disposée avec au moins un élément de fixation, configuré pour coopérer avec une suture pour suturer l'embase de distribution (210) à une structure à l'intérieur du corps.

14. Système comprenant :
- un cathéter (100) selon l'une quelconque des revendications 1 à 10, ou un ensemble cathéter (200) selon l'une quelconque des revendications 11 à 13,
- un réservoir contenant le liquide qui est une formule liquide thérapeutique, et
- un inducteur de flux configuré pour induire un flux de formulation liquide thérapeutique depuis le réservoir jusqu'au cathéter (100) ou l'ensemble cathéter (200).
